# EUROPEAN PATENT SPECIFICATION

(11) **EP 2 710 143 B1**
(45) Date of publication and mention of the grant of the patent: **16.03.2016**
(21) Application number: 12722150.5
(22) Date of filing: 18.05.2012
(51) Int. Cl.: C12Q 1/68

(54) **METHOD FOR DETERMINING WHETHER A SUBJECT IS AT RISK OF HAVING OR DEVELOPING A CHRONIC KIDNEY DISEASE**
VERFAHREN ZUR BESTIMMUNG DES RISIKOS EINES SUBJEKTS ZUR ENTWICKLUNG EINER CHRONISCHEN NIERENERKRANKUNG
MÉTHODE PERMETTANT DE DÉTERMINER SI UN SUJET RISQUE D'AVOIR OU DE DÉVELOPPER UNE INSUFFISANCE RÉNALE CHRONIQUE

(30) Priority: 18.05.2011 EP 11305601
(43) Date of publication of application: 26.03.2014
(73) Proprietor: INSERM (Institut National de la Santé et de la Recherche Médicale), 75013 Paris (FR); Assistance Publique - Hôpitaux de Paris, 75004 Paris (FR)
(72) Inventor: CHATZIANTONIOU, Christos, F-75020 Paris (FR); PLACIER, Sandrine, F-75020 Paris (FR); DUSSAULE, Jean-Claude, F-75020 Paris (FR)
(74) Representative: Holtz, Béatrice
(86) International application number: PCT/EP2012/059277
(87) International publication number: WO 2012/156513

(56) References cited:
- WO-A1-01/57062
- WO-A1-2008/089936
- WO-A2-2009/114699
- US-A1- 2011 177 613
- WALLACE DARREN P ET AL: "Periostin induces proliferation of human autosomal dominant polycystic kidney cells through alpha(V)-integrin receptor", AMERICAN JOURNAL OF PHYSIOLOGY - RENAL PHYSIOLOGY, vol. 295, no. 5, November 2008 (2008-11), pages F1463-F1471, XP002660072,
- DATABASE BIOSIS [Online] BIOSCIENCES INFORMATION SERVICE, PHILADELPHIA, PA, US; April 2009 (2009-04), MORRA LAURA ET AL: "Evaluation and functional characterization of periostin isoforms in lung and kidney carcinoma.", XP001537419, Database accession no. PREV200900497659 & PROCEEDINGS OF THE AMERICAN ASSOCIATION FOR CANCER RESEARCH ANNUAL MEETING, vol. 50, April 2009 (2009-04), page 1201, 100TH ANNUAL MEETING OF THE AMERICAN-ASSOCIATION-FOR-CANCER-RESEARCH; DENVER, CA, USA; APRIL 18 -22, 2009 ISSN: 0197-016X

## Description

### FIELD OF THE INVENTION:

The invention relates to the use of periostin as a biomarker of chronic kidney disease (CKD) in a patient. The invention thus relates to the identification of periostin as a marker for the diagnosis, in particular in urine sample or blood sample, and staging of CKD.

### BACKGROUND OF THE INVENTION:

Chronic kidney disease (CKD) is a major public health problem. Over the past years it has been learned that earlier identification and treatment of CKD can prevent kidney disease progression. Indeed, in France 60.000 patients are currently treated by dialysis or by transplantation (included 35.000 dialysed patients and 25.000 transplanted patients, representing 2% of the total expenditures of the healthcare system. Thus, the economic impact of CKD on the healthcare system poses challenges including a better understanding of the epidemiology of CKD even if a recent study published in 2008 by "Agence de la Biomédecine" (Réseau d'Epidémiologie et Information en Néphrologie) disclosed that the most common causes of CKD are hypertension (22,6%), diabetes (22%) and primary nephropathies (55%). It results that 3 millions of individuals would be affected with a CKD in France. Renal failure is known as a major cardiac risk factor. This has led to the definition of a new condition called cardiorenal syndrome (CRS) characterized by kidney failure and heart failure. The primarily failing organ may be either the heart or the kidney, and it is often this failing organ that precipitates the failure of the other.

Thus, a better understanding of the mechanisms underlying the aggravation of this disease is crucial for improving the patient's care and treatment. However until now, diagnosis of CKD is based on proteinuria and glomerular filtration rate (GFR) estimations but both indexes reach abnormal values when the disease is well advanced. There is still an existing need to develop a method for determining whether a subject is at risk of having or developing a CKD, notably during early-stage of CKD development.

Therefore, a biomarker of CKD that indicates the presence of both early damage and can be used to identify patients at an increased risk of progressive disease would favorably impact CKD diagnosis and treatment.

### SUMMARY OF THE INVENTION:

The present invention relates to a method for determining whether a subject is at risk of having or developing a chronic kidney disease (CKD) comprising determining the expression level of the periostin gene in a biological sample obtained from said subject.

The present invention also relates to a method for staging a CKD in a patient comprising determining the expression level of the periostin gene in a biological sample obtained from said patient.

The present invention further relates to a method for determining the responsiveness of a patient suffering from a CKD to a treatment comprising determining the expression level of the periostin gene in a biological sample obtained from said patient.

### DETAILED DESCRIPTION OF THE INVENTION:

The inventors made the observation that periostin may be used for an early and accurate detection of CKD as well as a correlation between the expression level of periostin gene and the severity of CKD (and therefore associated kidney lesions). Indeed, periostin is not detected in biological sample obtained from subjects and is detected in sample obtained from patients affected with a CKD. Furthermore, it has also been shown that the expression level of periostin gene is correlated with the severity of CKD and that the expression level of periostin gene is also useful to determine the responsiveness of a patient suffering from a CKD to a treatment (e.g. an antihypertensive treatment).

### Definitions:

Throughout the specification, several terms are employed and are defined in the following paragraphs.

The term "periostin" as used herein, is intended to encompass all synonyms including, but not limited to, "osteoblast specific factor 2", "OSF-2" or "POSTN".

The term thus includes naturally occurring periostin and variants and modified forms thereof. The term "periostin protein" refers to the periostin protein of 834 amino acids (provided in the GenPept database under accession number NP_006466.

Periostin is referred to as a "marker" or "biomarker" in that it enables to diagnose or predict the development of a chronic kidney disease (CKD).

The terms "biomarker", as used herein, refers generally to a molecule, i.e., a gene (or nucleic acid encoding said gene), protein, the expression of which in a biological sample from a patient can be detected by standard methods in the art (as well as those disclosed herein), and is predictive or denotes a condition of the patient from which it was obtained.

As used herein, the term "chronic kidney disease" (CKD) refers to a progressive loss in renal function over a period of months or years. CKD has its general meaning in the art and is used to classify numerous conditions that affect the kidney, destruction of the renal parenchyma and the loss of functional nephrons or glomeruli.

More precisely, Kidney Disease Improving Global Outcomes (KDIGO) developed and published for the first time a system for the definition and classification of stages of CKD.

Thus, CKD has been defined according to the criteria listed in according to the KDOQI CKD classification Table 11 (on the basis of reduction of glomerular filtration rate (GFR) in combination with signs of kidney damage as) (http://www.kidney.org/professionals/kdoqi/guidelines_ckd/Gif_File/kck_t11.gif)

**Table 11. Definition of Chronic Kideny Disease Criteria**

| |
|---|
| 1. Kidney damage for ≥ 3 months, as defined by structural or functional abnormalities of the kidney, with or without decreased GFR, manifest by *either:* |
| - Pathological abnormalities; or |
| - Markers of kidney damage, including abnormalities in the composition of the blood or urine, or abnormalities in imaging tests |
| 2. GFR<60mL/min/1.73 m² for ≥3 months, with or without kidney damage |

| |
|---|
| Methods to estimate GFR are disuse in Guideline 4. Markers of kidney damage are discussed in Guidelines 5-6. |

Examples of etiology of CKD include, but are not limited to hypertension, diabetes, glomerulonephritis, cardiovascular diseases, polycystic kidney diseases, and kidney graft rejection.

The term "patient" or "subject" as used herein denote a mammal. Preferably, a subject or a patient according to the invention is a human. The patient may be asymptomatic or may show early or advanced symptoms of CKD.

The term "healthy subjects" as used herein refers to a population of subjects who do not suffer from any known condition, and in particular, who are not affected with a CKD.

The term "biological sample" means any biological sample derived from a subject or a patient. Examples of such samples include fluids, tissues, cell samples, organs, biopsies, etc. Preferred biological samples are kidney biopsy. Other preferred biological samples are urine sample or blood sample.

By "blood sample" is meant a volume of whole blood or fraction thereof, eg, serum, plasma, etc.

### Diagnostic methods and kits:

The present invention relates to a method for determining whether a subject is at risk of having or developing a chronic kidney disease (CKD) comprising determining the expression level of the periostin gene in a biological sample obtained from said subject.

"Risk" in the context of the present invention, relates to the probability that an event will occur over a specific time period, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(1-p) where p is the probability of event and (1- p) is the probability of no event) to no- conversion.

In a particular embodiment, the methods of the invention further comprise a step consisting of comparing the determined expression level of periostin in the biological sample obtained from the subject with a reference level, wherein a difference between said determined expression level and said reference level is indicative whether said subject is at risk of having or developing a CKD.

As used herein, the term "reference level" refers to the amount of periostin in biological samples obtained from the general population or from a selected population of subjects. For example, the selected population may be comprised of apparently healthy subjects, such as individuals who have not previously had any sign or symptoms indicating the presence of CKD. In another example, the reference level may be of the amount of periostin obtained from subjects having an established CKD. The reference level can be a threshold value or a range. The reference level can be established based upon comparative measurements between apparently healthy subjects and subjects with established CKD.

In one embodiment, the reference value is derived from the expression level of periostin in a control sample derived from one or more subjects who are substantially healthy.

In another embodiment, such subjects are monitored and/or periodically retested for a diagnostically relevant period of time ("longitudinal studies") following such test to verify continued absence of CKD. Such period of time may be one year, two years, two to five years, five years, five to ten years, ten.years, or ten or more years from the initial testing date for determination of the reference value.

Furthermore, retrospective measurement of periostin levels in properly banked historical subject samples may be used in establishing these reference values, thus shortening the study time required, presuming the subjects have been appropriately followed during the intervening period through the intended horizon of the product claim.

Typically, the levels of periostin in a subject who is at risk for CKD is deemed to be higher than the reference value obtained from the general population or from healthy subjects.

The present invention also relates to a method for staging a chronic kidney disease (CKD) in a patient comprising determining the expression level of the periostin gene in a biological sample obtained from said patient.

In one embodiment, the patient is affected with a CKD selected in the group consisting of cardiovascular disease (such as e.g. hypertension), diabetes, glomerulonephritis (such as e.g. membranous glomerulonephritis and membranoproliferative glomerulonephritis), polycystic kidney disease (such as e.g. Autosomal Dominant Polycystic Kidney Disease (ADPKD) and Autosomal Recessive Polycystic Kidney Disease (ARPKD)), interstitial nephritis, lupus nephritis, nephropathy (such as e.g. membranous nephropathy), idiopathic nephrotic syndrome (such as e.g. Minimal Change Nephrotic Syndrome (MCNS) and Focal Segmental GlomeruloSclerosis (FSGS)), obstructive uropathy, and a kidney graft rejection (including acute and chronic kidney rejection).

A further aspect of the invention relates to a method for determining the responsiveness of a patient suffering from a CKD to a treatment comprising determining the expression level of the periostin gene in a biological sample obtained from said patient.

Typically said treatment may consist of administration of antihypertensive drugs or biotherapies (e.g. ACE inhibitors, renin inhibitors or AT1 receptor antagonists).

By "determining the responsiveness" of a subject to a treatment is meant evaluating the resolution or improvement of abnormal clinical features.

For example, in a subject suffering from CKD that responds to a treatment, a restoration of normal proteinuria can be observed. More specifically, "determining the responsiveness" of a subject to a treatment includes determining whether upon said treatment, the subject undergoes a complete remission, a partial remission, a remission with a high or a low risk of relapse, or whether said treatment will have no significant effect on the abnormal clinical features and/or the evolution of the disease.

In a particular embodiment, the method as above described further comprises the step of comparing the determined level of the periostin gene in a biological sample obtained from the patient with a reference level, wherein a difference between said determined level and said reference level is indicative whether said patient responds to the treatment. Typically, the reference value is derived from the level of periostin gene in a control sample derived from one or more patients who have responded or not responded to said treatment.

Methods for determining the level of a biomarker such as periostin gene or protein in a biological sample are well known in the art.

In one embodiment, determining the expression level of the periostin gene according to methods of the invention is performed by determining the quantity of mRNA encoding periostin in a biological sample obtained from said subject or patient.

Kidney biopsy is the preferred sample. Total RNAs can be easily extracted therefrom. The cell or tissue sample may be treated prior to its use, e.g. in order to render nucleic acids available. Techniques of cell or protein lysis, concentration or dilution of nucleic acids, are known by the skilled person.

Determination of the expression level of a gene can be performed by a variety of techniques. Generally, the expression level as determined is a relative expression level.

More preferably, the determination comprises contacting the sample with selective reagents such as probes, primers or ligands, and thereby detecting the presence, or measuring the amount of nucleic acids of interest originally in the sample.

In a preferred embodiment, the expression level may be determined by determining the quantity of mRNA.

Methods for determining the quantity of mRNA are well known in the art. For example the nucleic acid contained in the samples (e.g., biopsy prepared from the patient) is first extracted according to standard methods, for example using lytic enzymes or chemical solutions or extracted by nucleic-acid-binding resins following the manufacturer's instructions. The extracted mRNA is then detected by hybridization (e.g., Northern blot analysis) and/or amplification (e.g., RT-PCR).

In a preferred embodiment, the expression level of the periostin gene is determined by RT-PCR, preferably quantitative or semi-quantitative RT-PCR, even more preferably real-time quantitative or semi-quantitative RT-PCR.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization.

A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit.

Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another embodiment, determining the expression level of the periostin gene according to methods of the invention is performed by measuring the concentration of the periostin protein in a biological sample obtained from said subject or patient.

In a preferred embodiment, the concentration of the periostin protein is measured in a blood sample and/or urine sample obtained from said subject or patient. Once the biological sample from the patient is prepared, the concentration of periostin may be measured by any known method in the art.

In one embodiment, such methods comprise contacting the biological sample with a binding partner capable of selectively interacting with periostin present in the biological sample.

In one particular embodiment, the binding partner may be an antibody that may be polyclonal or monoclonal, preferably monoclonal.

In another particular embodiment, the binding partner may be an aptamer.

Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.

Monoclonal antibodies of the invention or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985).

Examples of said antibodies include, but are not limited to, the polyclonal antibody ab14041 from Abcam, polyclonal antibodies sc-67233 and sc-49480 from Santa-Cruz, the polyclonal antibody RD184045100 from Biovendor and monoclonal antibodies that specifically binds to human periostin described in the patent application WO 03/016471.

Alternatively, techniques described for the production of single chain antibodies (see e.g. U.S. Pat. No. 4,946,778) can be adapted to produce anti-periostin, single chain antibodies. Antibodies useful in practicing the present invention also include anti-periostin fragments including but not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed to allow rapid identification of fragments having the desired specificity to periostin. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.

In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996).

The binding partners of the invention such as antibodies or aptamers may be labelled with a detectable molecule or substance, such as a fluorescent molecule, an enzyme able to produce a coloured product, a radioactive molecule or any others labels known in the art. Labels are known in the art that generally provide (either directly or indirectly) a signal.

As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or indocyanine (Cy5), to the antibody or aptamer, as well as indirect labelling of the probe or antibody (e.g., horseradish peroxidise, HRP) by reactivity with a detectable substance. An antibody or aptamer of the invention may be labelled with a radioactive molecule by any method known in the art. For example radioactive molecules include but are not limited radioactive atom for scintigraphic studies such as 1123,1124, In111 Rel86, Re188.

The aforementioned assays generally involve the binding of the binding partner (i.e., antibody or aptamer) in a solid support. Solid supports which can be used in the practice of the invention include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of anti-periostin antibodies. A biological sample containing or suspected of containing periostin is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art. An alternative method of ELISA involves the addition of a biological sample containing or suspected of containing periostin to the coated wells in addition to a known amount of labelled periostin. After a period of incubation sufficient to allow the formation of antibody-antigen complexes and the competition between labelled and non-labelled naturally occurring molecules, the plate(s) can be washed to remove unbound moieties. The intensity of the label obtained by the binding of, for example HRP-conjugated periostin, is detected using methods well known in the art. In this configuration, that is more sensitive that common ELISA, decrease of the label is in relation with the amount of natural molecules present in the sample. In the case of HRP-conjugated periostin, the addition of a HRP substrate will allow the detection. Colour detection is commonly used but needs incubation time, whereas luminol-derived substances alternatives allow a direct (no incubation time) and a highly sensitive detection.

The concentration of periostin may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation.

Measuring the concentration of periostin (with or without immunoassay-based methods) may also include separation of the compounds: HPLC based on hydrophobicity; size exclusion chromatography based on size; and solid-phase affinity based on the compound's affinity for the particular solid-phase that is used. Once separated, periostin may be identified based on the known "separation profile" e. g., retention time, for that compound and measured using standard techniques.

Alternatively, the separated compounds may be detected and measured by, for example, a mass spectrometer.

A further aspect of the invention relates to the use of periostin as a biomarker of a CKD in a patient.

A further aspect of the invention relates to the use of a kit detecting periostin for diagnosing CKD in a patient.

The invention will be further illustrated by the following figures and examples. However, these examples and figures should not be interpreted in any way as limiting the scope of the present invention.

### FIGURES:

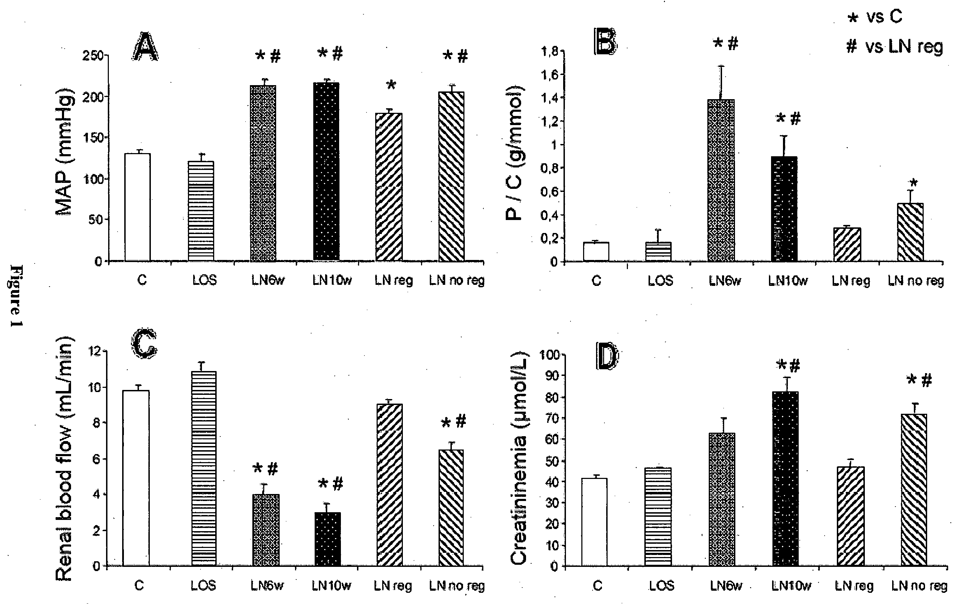

**Figure 1****:** Mean arterial pressure (A) proteinuria (B), renal blood flow (C) and plasma creatinine (D) in control rats (C) and rats treated with losartan (LOS), L-NAME 6 weeks (LN6w), L-NAME 10 weeks (LN10w), L-NAME+losartan which regressed (LN reg) and L-NAME+losartan which escape therapy (LN no reg). The exact description of the treatments in each group is provided in methods below.

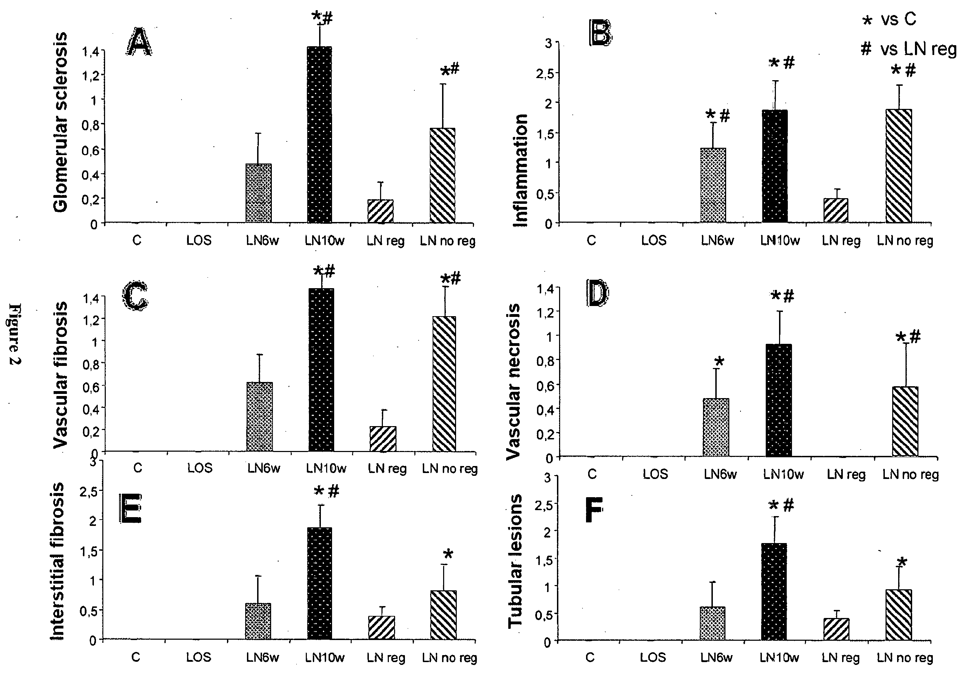

**Figure 2****:** Quantification of lesions in the renal cortex of control rats (C) and rats treated with losartan (LOS), L-NAME 6 weeks (LN6w), L-NAME 10 weeks (LN10w) L-NAME+losartan which regressed (LN reg) and L-NAME+losartan which escaped therapy (LN no reg). A: Glomerular sclerosis; B: Total Inflammation; C: Vascular fibrosis; D: vascular necrosis; E: Interstitial fibrosis; F: Tubular lesions.

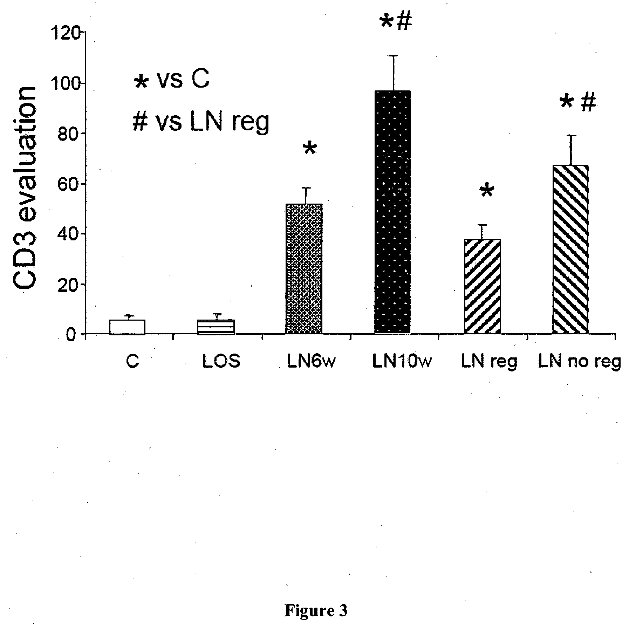

**Figure 3****:** Quantification of the T-lymphocytes presence (as evidenced by CD3 staining) in the renal cortex of control rats (C) and rats treated with losartan (LOS), L-NAME 6 weeks (LN6w), L-NAME 10 weeks (LN10w)_{;} L-NAME+losartan which regressed (LN reg) and L-NAME+losartan which escaped therapy (LN no reg).

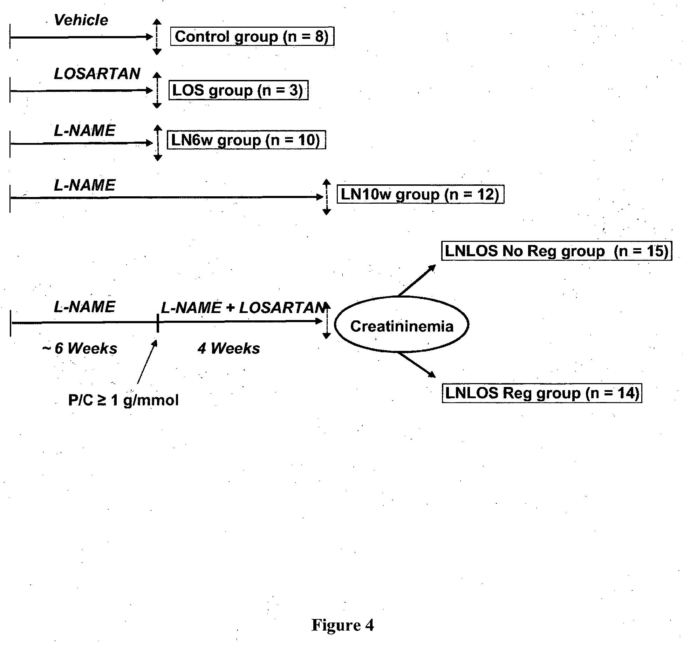

**Figure 4****:** Experimental protocol showing the treatment and the different groups used in the study of hypertension-induced CKD. For the analytical description of the treatments please refer to the Material & Methods description (animal treatment) below.

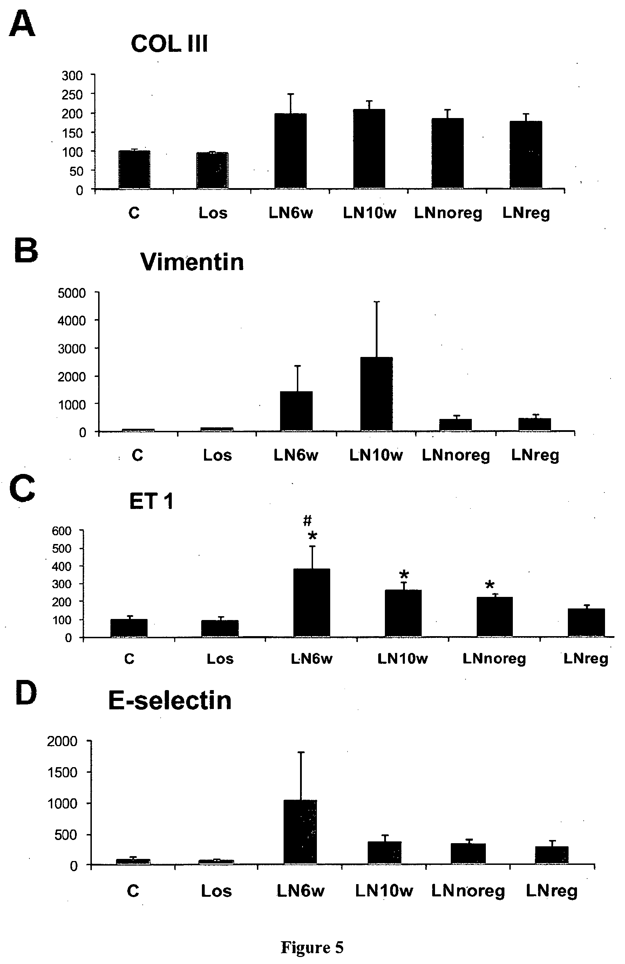

**Figure 5****:** mRNA expressions of collagen III (A), vimentin (B), endothelin (ET-1) (C), e-selectin (D) in the renal cortex of control rats (C) and rats treated with losartan (Los), L-NAME 6 weeks (LN6w), L-NAME 10 weeks (LN10w), L-NAME+losartan which regressed (LN reg) and L-NAME+losartan which escaped therapy (LN no reg). Note that e-selectin is highly expressed early, but its expression decreased afterwards.

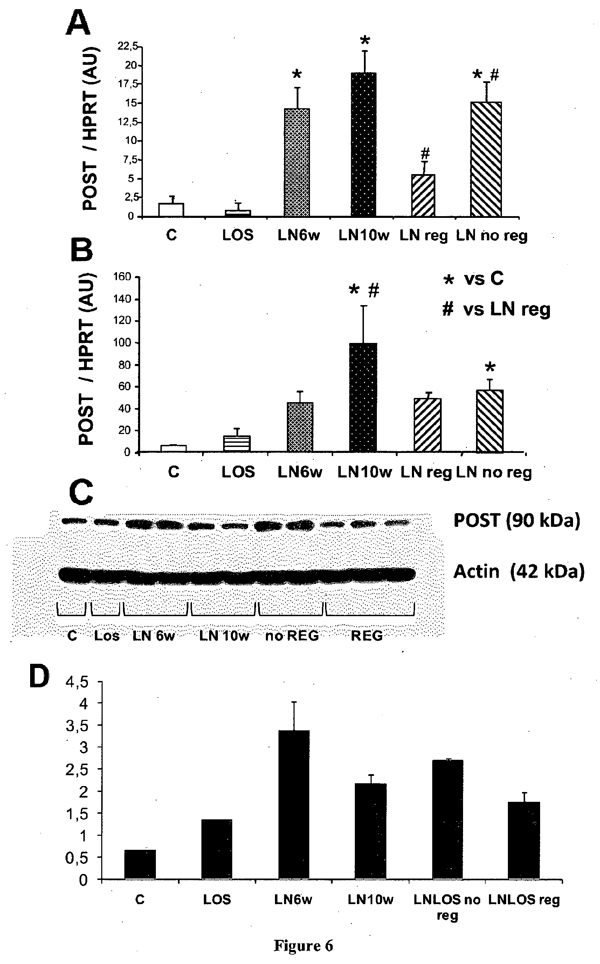

**Figure 6****:** mRNA expressions of periostin in the renal cortex (A) and aorta (B) of control rats (C) and rats treated with losartan (Los), L-NAME 6 weeks (LN6w), L-NAME 10 weeks (LN10w), L-NAME+losartan which regressed (LN reg) and L-NAME+losartan which escaped therapy (LN no reg). Note that periostin expression in the renal cortex varies according to the degree of the renal disease in the different groups.6C: Representative examples of periostin expression quantified by western blot control rats (C) and rats treated with losartan (Los), L-NAME 6 weeks (LN6w), L-NAME 10 weeks (LN10w), L-NAME+losartan which regressed (LN reg) and L-NAME+losartan which escaped therapy (LN no reg). 6D: Quantitative estimation by densitometry of the bands observed in the western blots of 6C panel.

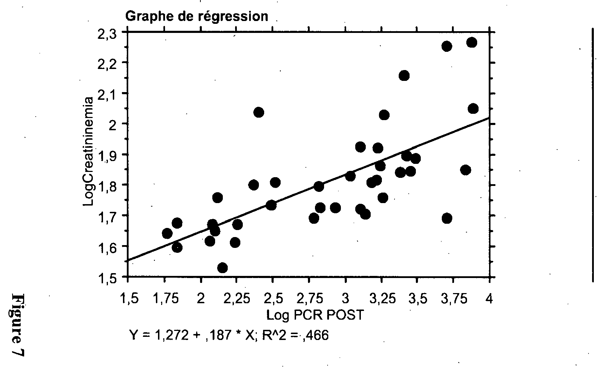

**Figure 7****:** A: Interactions between periostin expression and creatininemia in animals.

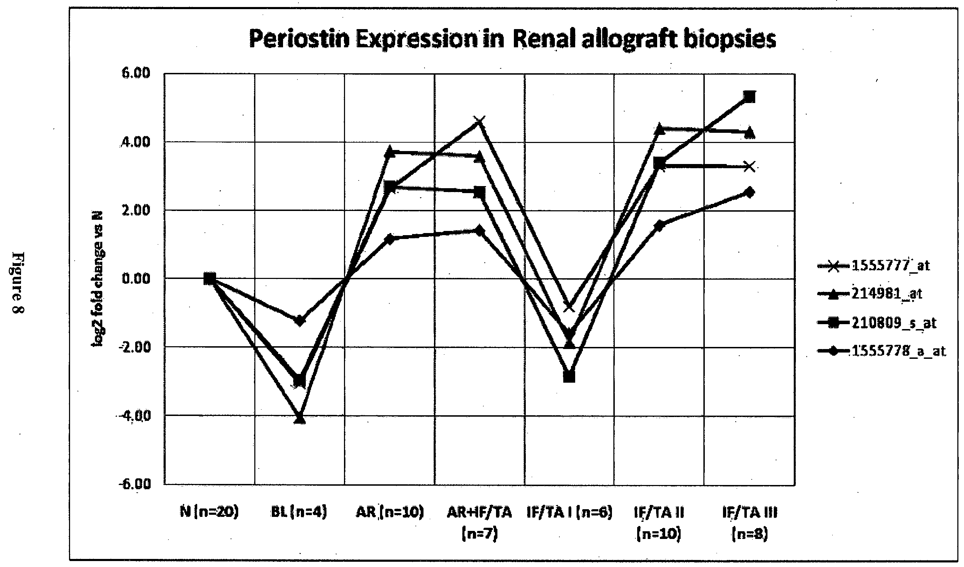

**Figure 8****:** Transcriptomic analysis of periostin expression in renal graft biopsies.

### EXAMPLE 1: Hypertension-induced renal disease rat model (L-NAME)

### Material & Methods

### Groups:

C (control rats): n=8
LOS (rats treated with losartan): n=3
LN6w (rats treated with L-NAME and killed at 6 weeks): n= 10
LN10w (rats treated with L-NAME and killed at 10 weeks): n= 12
LN reg (rats treated with L-NAME+losartan which regressed): n=14
LN no reg (rats treated with L-NAME+losartan which escaped therapy): n=15

***Animal treatment:*** Male Sprague-Dawley rats, weighing 250 g, were maintained on a normal-salt diet and had free access to chow and tap water. NO synthesis was inhibited by L-NAME (orally, 15 mg•kg-1•day-1). We have previously found that this dose produced a gradual elevation of blood pressure accompanied by the progression of renal disease. When proteinuria exceeded 1 g/mmol creatinine (after 5 or 6 weeks), a group of animals was killed to allow estimations of renal hemodynamics and morphological parameters just before the beginning of therapy (LN 6w group, n = 10). The remaining animals were divided into two subgroups for an additional experimental period of 4 weeks: in the first subgroup, L-NAME was given alone (LN 10w group, n = 12); in the second subgroup treatment was accompanied by the administration of an AT1 receptor antagonist (Losartan, orally 30 mg•kg-1•day-1, Merck Sharp and Dohme-Chibret). At the end of Losartan treatment, we separated the animals of the second group in two subgroups: animals with normal creatininemia below 50 µmol/l (LN reg n= 14) and animals with a creatininemia above 70 µmol/l (LN no reg n= 15). The doses of the drugs were based on pilot experiments and previously published studies. Control animals were sacrificed at 6 and 10 week. Since control animals gave similar results for all measured parameters, pooled data are presented (C group, n = 8). Others animals received Losartan during 4 weeks as additional control group (LOS group, n= 3). All protocols and treatments were performed with the approval of the French government ethics committee.

***Renal hemodynamics:*** After anesthesia by pentobarbital sodium (50-60 mg/kg body wt ip, Nembutal, Abbott, Chicago, IL), animals were placed on a servo-controlled table kept at 37°C and the trachea was cannulated to facilitate respiration. The left femoral artery was catheterized for measurement of arterial pressure, and a femoral venous catheter was used for infusion of volume replacement. An ultrasound transit-time flow probe (1RB, Transonic, Ithaca, NY) was placed around the left renal artery. Bovine serum albumin (4.7 g/dl of saline solution) was infused initially at 50 µl/min to replace surgical losses, and then at 10 µl/min for maintenance. Arterial pressure was measured via a pressure transducer (Statham P23 DB); RBF was measured by a flowmeter (T 420, low-pass filter, 40 Hz, Transonic). RBF values were controlled for zero offset determined at the end of an experiment after cardiac arrest. Data were recorded, stored, and analyzed using a DataTranslation analog-to-digital converter and the IOX software (EMKA Technologies, Paris, France).

***Urinary protein excretion and plasma creatinine:*** Urine samples were collected for a 4-h period. Urinary protein concentration was normalized to creatinine concentration, and values were expressed as grams protein per millimole creatinine. Blood samples were withdrawn on the last day of the study, and plasma creatinine (µmol/l) was measured by automated Jaffe's method.

***Renal histology:*** Kidneys were stained with Masson's trichromic solution. Sections of kidneys were examined on a blinded basis by two investigators independently to estimate inflammation, tubular lesions, interstitial fibrosis, vascular fibrosis, glomerular sclerosis and vascular necrosis using a 0 to 4 injury scale as described previously. Lesion indexes from individual sections were averaged to calculate a sclerotic index for each rat.

***Immunohistochemistry for CD3:*** Four-micrometer-thick sections of paraffin-embedded kidneys were dewaxed, heated in citric acid solution (pH6) at 98°C for 30 min, and incubated first with a polyclonal goat anti-rat CD3 antibody recognizing lymphocytes (Santa Cruz Biotechnology, Santa Cruz, CA) for two hours at 37°C and then incubated for 30 min at room temperature with a second antibody from N-Histofine kit (Nichirei Biochemicals, Japan). Staining was revealed by applying AEC (Dako), counterstained with hematoxylin QS (Vector, Burlingame, CA), and finalized with Permanent Aqueous Mounting Media (Innovex). Quantification of CD3-positive cells was performed using Olympus analysis software.

***Immunohistochemistry for Periostin (POST):*** Four-micrometer-thick of frozen sections were incubated with polyclonal rabbit anti-rat POST antibody (Abcam, Cambridge, UK) overnight at 4°C and then incubated for 30 min at room temperature with a second antibody from N-Histofine kit (Nichirei Biochemicals, Japan). Staining was revealed by applying AEC (Dako), counterstained with hematoxylin QS (Vector, Burlingame, CA), and finalized with Permanent Aqueous Mounting Media (Innovex).

***Real Time quantitative PCR:*** We extracted RNA from the renal cortex and the abdominal aorta using TRIzol solution (Life Technologies BRL, Gaithersburg, MD). RNA quality was checked by measuring the ratio of optical densities at 260 and 280 nm and residual genomic DNA was removed by DNase I treatment for 30 min at 37°C (Fermentas). We used reverse transcription with Revert Aid H minus First Strand DNA Synthesis kit (Fermentas) to convert 1 µg RNA into cDNA, which was then amplified by PCR using a LightCycler 480 (Roche Diagnostic) using SYBR Green (Fast Start DNA Master SYBRGreen I; Roche Applied Science, Roche Diagnostic), specific primers for POST, Col3A1, ESel, Vimentin, VCAM-1 and hypoxanthine-guanine phosphoribosyltransferase (HPRT) as housekeeping gene under the following conditions: 95°C for 5 min, and 45 cycles at 95°C for 15 s and 60°C for 15 s, then 72°C for 15 s. Specific primers were designed by Universal Probe Library system (UPL, Roche Applied Science). To normalize the Q-PCR results we used Roche LightCycler 2.0 software (Roche Diagnostic). We expressed results as 2-deltaCp, where Cp is the cycle threshold number. We analyzed dissociation curves after each run for each amplicon to access the specificity of quantification when using SYBR Green.

***Immunoblotting of POST in renal cortex:*** 25 µg of protein extracts obtained by RIPA buffer lysis of renal cortex were fractionated by SDS-PAGE NuPAGE 4/12% gels (Invitrogen) under reducing conditions using an XCell SureLockTM Mini-Cell (NuPAGE, Invitrogen, San Diego, CA, USA) as described by the manufacturer and transferred onto a nitrocellulose membrane (Immobilon-P, Millipore, Billerica, MA, USA) prior to detection of the POST (Biovendor) or β-actin (Imgenex, Clinisciences, France) protein with a specific rabbit polyclonal primary Ab (dilution 1/1000 and 1/5000 respectively) and a peroxidase-labelled IgG anti-rabbit secondary Ab (dilution 1/4000). The membrane was detected by enhanced chemiluminescence (ECL)-plus kit (Amersham Biosciences, Piscataway, NJ, USA) on autoradiography films (Fuji).

***Statistical methods:*** Statistical analyses for the in vivo studies were performed using ANOVA followed by Fisher's protected least significance difference test in the Statview software package. All values are means ± SE. Associations between periostin mRNA expression and selected functional and histological variables were tested by linear regressions, after log transformation for non-normally distributed variables. Results with P < 0.05 were considered statistically significant.

### Results

***Pharmacological nitric oxide inhibition induces progressive renal vascular disease:*** After initiation of LNAME treatment rats rapidly developed severe persistent hypertension (MAP = 211±7 mmHg, and 212±5 mmHg at 6 and 10 weeks treatment respectively) (Figure 1A). Progressive hypertensive renal disease was characterized by the early onset of proteinuria (1.3±0.2 g/mmol creatininuria at week 6) and a delayed increase in creatininemia (100±14 µmol/l at week 10) (Figure 1B, 1D). In parallel, renal blood flow exhibited a striking reduction, from 6 weeks LNAME treatment onwards (Figure 1C).

As expected, these functional alterations were associated with progressive histological lesions of vascular nephropathy (Figure 2) including glomerulosclerosis (Figure 2A), vascular fibrosis (Figure 2C), vascular necrosis (Figure 2D), interstitial fibrosis (Figure 2E), tubular lesions (Figure 2F) and inflammation (Figure 2B). A characterization of the inflammatory infiltrating cells showed that CD3+ lymphocyte count was strongly increased after 6 and 10 weeks LNAME treatment.

***Losartan promotes regression of renal disease in a subpopulation of LNAME-treated rats:*** To study determinants of renal disease progression/regression, rats with mild hypertensive nephropathy (proteinuria/creatininuria - 1g/mmol) were treated with Losartan, together with the continuation of LNAME. Important heterogeneity in the evolution of renal disease was observed, when compared to the similar degree of proteinuria at the initiation of Losartan. Rats were thereafter separated in two groups according to the median value of creatininemia after 4 weeks Losartan + LNAME treatment (Figure 4). Accordingly, the "No Regression" (NOREG) group presented a significantly higher creatininemia compared to the "Regression" group (REG) (76±3 vs 50±2 µmol/l, p<0.001). Consistent with the differential therapeutic efficiency of Losartan between the 2 groups, NOREG rats exhibited aggravated renal blood flow (Figure 1C), vascular lesions (Figure 2C and 2D), glomerulosclerosis (Figure 2A) and renal inflammation (Figures 2B and 4) compared to REG group.

***Characterization of markers associated with different stages of hypertensive renal disease:*** NO inhibition is associated with the accumulation of extracellular matrix in the kidney. Although significant fibrosis was detected on week 10, but not on week 6 (Figure 2), Col3A1 RT-qPCR was already up-regulated on week 6, indicating persistently increased synthesis of collagen from this stage onwards (Figure 5A). Note that Col3A1 synthesis was not different between REG and NOREG groups.

Vimentin was progressively induced at week 6 and week 10 (Figure 5B). In contrast to Col3A1, vimentin exhibited a sharp decrease after the initiation of losartan, irrespective of the REG / NOREG group.

Since endothelial injury may play a central role in the pathophysiology of renal vascular diseases, we investigated the transcriptional regulation of ET-1 propeptide and E-selectin in this model (Figure 5C and 5D). Interestingly, both markers were presented a strong but transient induction at week 6, suggesting early endothelial dysfunction. However, neither E-selectin, nor ET-1 enabled to discriminate REG group from NOREG group during the progression of the disease.

***Periostin is closely associated with the severity of hypertensive nephropathy and is a major determinant of kidney disease regression:*** As shown by RT-qPCR, periostin was expressed in the kidney at baseline and was 13- and 18-fold up-regulated after 6 and 10 weeks LNAME respectively (Figure 6A). Importantly, periostin mRNA and protein were significantly blunted in REG group (Figure 6A), whereas NOREG group presented a persistent increase in periostin expression in spite of 4 weeks losartan treatment (Figure 6,). The latter difference of periostin expression observed between REG and NOREG groups was absent when evaluated in the aorta (Figure 6B). Quantitiative evaluation of periostin protein expression by western blotting confirmed the above-described transcriptional differences (Figure 6C, 6D)

Immunohistochemistry revealed that periostin presented a weak expression in the normal kidney, within the media of arteries and arterioles. Hypertensive nephropathy was characterized by a strong increase in periostin staining in the media and the adventitia of renal vessels. Interestingly periostin also exhibited a focal de novo interstitial expression in close vicinity to the most severe vascular, glomerular and tubular lesions.

To further evaluate the importance of periostin as a marker and/or an actor of kidney disease progression in hypertension, we performed regression analyses with classical functional and histological features of hypertensive nephropathy. We found a very strong association between the degree of periostin mRNA expression and creatininemia (r= 0.68 Figure 7.

### EXAMPLE 2: Transplantation (acute and chronic kidney graft rejection)

### Material & Methods

Renal biopsies from renal transplanted patients (acute and chronic kidney graft rejection) have been obtained.

### Results

The main observations obtained in the field of transplantation as shown in Figure 8 are:
1) Results are similar independently of the pair of the primers used, eliminating thus the probability of a non-specific amplification;
2) Periostin is increased in biopsies from acute rejection patients (AR group); this increase is amplified when inflammatory lesions are observed (group AR+IF);
3) The involvement of periostin is observed also in chronic rejection biopsies. The expression of periostin is increasing with and is associated to the degree of structural lesions (groups TA I vs TA II vs TA III).

To determine whether the latter findings may translate to human disease, we further analyzed periostin immunostaining on human kidney biopsy specimens. Periostin was not expressed in glomeruli or tubules in normal renal tissue. Only a weak staining could be found in small vessels. Chronic allograft nephropathy is a clinical condition characterized by tubulointerstitial inflammation and fibrosis. In this condition we observed an intense periostin expression, predominantly in the region presenting tubular atrophy and interstitial fibrosis, as well as in several tubular epithelial cells. Using serial sections with periostin and vimentin, the latter indicating epithelial phenotypic changes associated with renal graft fibrosis progression, the expression of periostin was mainly located in the interstitial area around the injured tubules, suggesting the importance of periostin expression during human kidney injury.

### DISCUSSION:

The renin-angiotensin system is a central player in multiple mechanisms responsible for the progression of renal fibrosis. Timely blockade with ACE inhibitors, renin inhibitors or AT1 receptor antagonists can promote reversal of renal lesions and ultimately prevent the evolution towards end-stage organ failure in experimental models of renal disease. However, renin-angiotensin system blockers are inconstantly efficient in preventing chronic renal disease progression, particularly in humans, although the factors involved in this variable efficacy remain unclear.

In this study we analyzed the reversal of L-NAME hypertensive nephropathy with losartan, and focused on the identification of markers of a non-return point of renal disease reversal. Our results show that losartan ameliorated renal hemodynamic alterations, proteinuria, and vimentin expression in all groups treated, compared to L-NAME 6w and 10w groups. Since these variables are classical determinants of renal disease progression, it could have been expected that the latter effects would be associated with a global protection against the functional and structural alterations ultimately induced by NO-deficiency hypertension. Instead, in spite of these beneficial effects, the No Reg group presented severe functional and structural disease, characterized by elevated plasma creatinine and vascular fibrosis similar to LN 10w group. Important additional factors implicated in renal disease progression and influenced by losartan are therefore responsible for the differential evolution between the Reg and No Reg groups. The reason why these important factors were different between the two groups is uncertain. In spite of the standardized cut-off point chosen to introduce losartan, we cannot exclude that the heterogeneity observed between Reg and No Reg groups may be due to subtle differences in the evolution of the renal disease around the non-return point, when losartan was introduced. Alternatively, preexisting heterogeneity in the regulation of pro- or anti-fibrotic genes between the Sprague Dawley rats, an outbred strain in which genome is not totally identical between animals, could account for the differences between the Reg and No Reg groups. We took advantage of these original experimental conditions to perform a transcriptomic analysis of selected markers associated with the progression of hypertensive nephropathy.

In the Reg group, rats presented reduced renal fibrosis compared to the No Reg group. Renal fibrosis is characterized by the accumulation of extracellular matrix, including fibrillar collagen. Since collagen III production, evaluated by Col3A1 RT-qPCR, was not different between the Reg and No Reg groups, the histological differences observed may be due to increased degradation of fibrillar collagen.

L-NAME hypertension is associated with features of endothelial dysfunction. Our results show early endothelial activation indicated by increased expression of ET-1 propeptide and E-selectin in LN 6w group. We and others have previously identified ET-1, a potent profibrotic vasoconstrictor, as a mediator of renal fibrosis in hypertensive nephropathy. E-selectin is an endothelial inducible adhesion molecule notably involved in the pathophysiology of atherosclerosis and in renal ischemia-reperfusion, but its potential implication in hypertensive nephropathy has not been reported previously. Although the present study shows early increase in renal ET-1 and E-selectin mRNA, their similar expressions in Reg and No Reg groups suggest that these markers of endothelial activation are not major determinants of the reversal of renal disease induced by AT1 receptor blockade. Similarly, vimentin, a classical marker of mesenchymal cells associated with fibrogenesis, was reduced by losartan treatment, irrespective of the reversal of renal disease.

Periostin is an extracellular matrix protein first identified in the periosteum and the periodontal ligament. Angiotensin II can induce periostin expression in fibroblasts and vascular smooth muscle cells, via Ras/p38 MAPK/CREB and ERK1/2/TGF-beta1 pathways and via phosphatidylinositol-3-kinase signaling, respectively. Accordingly, periostin is induced in models of ischemic, hypertensive and hypertrophic cardiomyopathies, and an AT1 receptor antagonist decreases the cardiac expression of periostin. In the kidney, experimental studies evaluating the implication of periostin in physiology and disease are scarce. Periostin is transiently expressed during renal development and the expression in the normal adult kidney is low. Whether periostin is implicated in hypertensive nephropathy and in the progression/reversal of chronic kidney disease remained unknown.

Our results identified a progressive induction of periostin in the kidney with the progression of the hypertensive nephropathy. Regression analyses found a strong association between periostin mRNA expression and robust functional markers of kidney disease. Importantly, these associations held true when systolic blood pressure was added as a covariate, which suggests that periostin is correlated to renal injury independently of the degree of hypertension. Immunohistochemical analyses revealed that the localization of periostin was predominantly perivascular, in areas where important deposits of extracellular matrix occur in this model. We also observed an intense predominantly extracellular staining for periostin in the injured fibrotic tubulo-interstitial regions of chronic allograft nephropathy, which further demonstrates overexpression of periostin in human kidney disease. Identification of the main cells responsible for the interstitial accumulation of periostin requires further investigation. Data from previous studies suggests that fibroblasts, smooth muscle cells and tubular epithelial cells may be involved in periostin expression in this setting.

We found that after the onset of hypertensive renal disease, curative treatment with losartan was associated with diminished periostin expression in Reg, not in No Reg group, which suggests that the reduction of periostin may be implicated in the mechanisms of angiotensin II-related disease progression and that reduction of periostin expression may be a critical determinant of disease reversal. Interestingly, the analysis of histological fibrosis scores shows that the difference between Reg and No Reg groups was most evident for perivascular fibrosis, in accordance with periostin distribution in experimental hypertensive nephropathy. These original data suggest that periostin may be related to the pathophysiology of extracellular matrix accumulation at the site of renal injury.

Together, the results of this work identify periostin as a previously unrecognized marker associated with hypertensive nephropathy. Further research is necessary to precise the potential of renal, plasma and urine periostin as prognostic biomarkers to monitor the progression and therapeutic control of human chronic kidney diseases.

## Claims

1. A method for determining whether a subject is at risk of having or developing a chronic kidney disease (CKD) comprising determining the expression level of the periostin gene in a biological sample obtained from said subject, wherein said CKD is a hypertensive nephropathy or a chronic allograft nephropathy.

2. The method according to claim 1, which further comprises a step consisting of comparing the determined expression level of periostin gene in the biological sample obtained from the subject with a reference level, wherein a difference between said determined expression level and said reference level is indicative whether said subject is at risk of having or developing a CKD.

3. A method for determining the responsiveness of a patient suffering from a hypertensive nephropathy to an antihypertensive drug treatment comprising determining the expression level of the periostin gene in a biological sample obtained from said patient and further comparing the determined level of the periostin gene in a biological sample obtained from the patient with a reference level, wherein a decrease between said determined level and said reference level is indicative of whether said patient responds to the treatment.

4. The method according to any one of claims 1 to 3, wherein said expression level of the periostin gene is determined by measuring the quantity of the periostin mRNA and said biological sample is a tissue sample.

5. The method according to claim 4, wherein said tissue sample is a kidney biopsy.

6. The method according to any one of claims 1 to 3, wherein said expression level of the periostin gene is determined by measuring the concentration of the periostin protein in a biological sample obtained from said patient.

7. The method according to claim 6, wherein said biological sample is an urine sample or blood sample.

8. Use of periostin as a biomarker of CKD in a patient, wherein said CKD is a hypertensive nephropathy or a chronic allograft nephropathy

## Patentansprüche

1. Verfahren zur Bestimmung, ob ein Subjekt gefährdet ist, ein chronisches Nierenleiden (CKD) zu haben oder zu entwickeln, umfassend ein Bestimmen des Expressionsspiegels des Periostin-Gens in einer biologischen Probe, die vom Subjekt erhalten wird, wobei das CKD eine hypertone Nephropathie oder eine chronische Homotransplantat-Nephropathie ist.

2. Verfahren nach Anspruch 1, welches ferner einen Schritt umfasst, der aus dem Vergleichen des bestimmten Expressionsspiegels des Periostin-Gens in der vom Subjekt erhaltenen biologischen Probe mit einem Referenzspiegel besteht, wobei eine Differenz zwischen dem bestimmten Expressionsspiegel und dem Referenzspiegel anzeigt, ob das Subjekt gefährdet ist, ein CDK zu haben oder zu entwickeln.

3. Verfahren zur Bestimmung der Ansprechempfindlichkeit eines an hypertoner Nephropathie leidenden Patienten für eine hypertonussenkende Arzneimittelbehandlung, umfassend ein Bestimmen des Expressionsspiegel des Periostin-Gens in einer vom Patienten erhaltenen biologischen Probe und ferner Vergleichen des bestimmten Spiegels des Periostin-Gens in einer vom Patienten erhaltenen biologischen Probe mit einem Referenzspiegel, wobei eine Senkung zwischen dem bestimmten Spiegel und dem Referenzspiegel anzeigt, ob der Patient auf die Behandlung anspricht.

4. Verfahren nach einem der Ansprüche 1 bis 3, wobei der Expressionsspiegel des Periostin-Gens durch Messen der Menge der Periostin-rnRNA bestimmt wird, und die biologische Probe eine Gewebeprobe ist.

5. Verfahren nach Anspruch 4, wobei die Gewebeprobe eine Nierenbiopsie ist.

6. Verfahren nach einem der Ansprüche 1 bis 3, wobei der Expressionsspiegel des Periostin-Gens durch Messen der Konzentration des Periostin-Proteins in einer vom Patienten erhaltenen biologischen Probe bestimmt wird.

7. Verfahren nach Anspruch 6, wobei die biologische Probe eine Urinprobe oder eine Blutprobe ist.

8. Verwendung von Periostin als Biomarker von CKD in einem Patienten, wobei das CKD eine hypertone Nephropathie oder eine chronische Homotransplantat- Nephropathie ist.

## Revendications

1. Procédé pour déterminer si un sujet est exposé à un risque de présenter ou de développer une maladie rénale chronique (MRC), comprenant la détermination du taux d'expression du gène de la périostine dans un échantillon biologique obtenu à partir dudit sujet, où ladite MRC est une néphropathie hypertensive ou une néphropathie chronique d'allogreffe.

2. Procédé selon la revendication 1, qui comprend en outre une étape consistant à comparer le taux d'expression déterminé du gène de la périostine dans l'échantillon biologique obtenu à partir du sujet avec un taux de référence, où une différence entre ledit taux d'expression déterminé et ledit taux de référence indique si ledit sujet est exposé à un risque de présenter ou de développer une MRC.

3. Procédé pour déterminer la réponse d'un patient souffrant d'une néphropathie hypertensive à un traitement par un médicament antihypertenseur, comprenant la détermination du taux d'expression du gène de la périostine dans un échantillon biologique obtenu à partir dudit patient et en outre la comparaison du taux déterminé du gène de la périostine dans un échantillon biologique obtenu à partir du patient avec un taux de référence, où une réduction entre ledit taux déterminé et ledit taux de référence indique si ledit patient répond au traitement.

4. Procédé selon l'une quelconque des revendications 1 à 3, dans lequel ledit taux d'expression du gène de la périostine est déterminé en mesurant la quantité d'ARNm de la périostine et ledit échantillon biologique est un échantillon de tissu.

5. Procédé selon la revendication 4, dans lequel ledit échantillon de tissu est une biopsie rénale.

6. Procédé selon l'une quelconque des revendications 1 à 3, dans lequel ledit taux d'expression du gène de la périostine est déterminé en mesurant la concentration de la protéine périostine dans un échantillon biologique obtenu à partir dudit patient.

7. Procédé selon la revendication 6, dans lequel ledit échantillon biologique est un échantillon d'urine ou un échantillon de sang.

8. Utilisation de périostine en tant que biomarqueur d'une MRC chez un patient, où ladite MRC est une néphropathie hypertensive ou une néphropathie chronique d'allogreffe.
